# EUROPEAN PATENT APPLICATION

(11) **EP 4 026 419 A1**
(43) Date of publication of application: **13.07.2022**
(21) Application number: 21213279.9
(22) Date of filing: 09.12.2021
(51) Int. Cl.: A01D 43/08, A01D 43/10

(54) **HARVESTER COMPRISING A PROCESSOR ROLL GAP CONTROL USING CROP MOISTURE CONTENT**

(30) Priority: 12.01.2021 US 202117146596
(71) Applicant: Deere & Company, Moline, IL 61265 (US)
(72) Inventor: Franzen, Devin M., 68163 Mannheim (DE); Murray, Cole L., 68163 Mannheim (DE)
(74) Representative: Holst, Sönke

(57) **Abstract**

A harvester includes a crop processor operable to process a crop material to alter a characteristic of the crop material, such as a percent fracture of a kernel. A moisture sensor is operable to sense data related to a moisture content of the crop material. A computing device receives the data from the moisture sensor and determines an actual moisture content of the crop material. The computing device may then adjust the crop processor based on the actual moisture content of the crop material to achieve a desired level of alteration to the characteristic of the crop material. For example, the computing device may adjust a roll gap of the kernel processor based on the moisture content of the crop material.

## Description

### TECHNICAL FIELD

The disclosure generally relates to a harvester having a crop processor for processing crop material, and a method of controlling the harvester.

### BACKGROUND

A harvester gathers crop material from a field and directs the crop material through a pair of opposing feed rollers. The crop material is fed between the opposing feed rollers, which move the crop material along a processing flow path. The feed rollers counter-rotate relative to each other to move the crop material in a direction of crop processing, which is generally rearward relative to a direction of travel of the harvester. Crop processing operations may include one or more post collection operations that improves the digestibility of the crop material, thereby increasing nutrient value of the crop material when consumed by animals.

The crop processing operations may include, but are not limited to, cutting the crop material to a length and/or fracturing/cracking kernels of the crop material. For example, the crop material may move along the flow path through a cutter head. The cutter head includes a rotating drum with a plurality of knives disposed on the periphery of the drum. The cutter head cooperates with a shear bar to cut stem portions of the crop material into small pieces. Following the cutter head, the crop material may flow through a kernel processor. The kernel processor includes a pair of processing rolls spaced apart from each other by a roll gap. Kernels in the crop material are fractured, i.e., cracked, as they move between the pair of processing rolls. The kernels include an outer shell or hull, which acts as a barrier against digestion by an animal. The hull of the kernel must be broken. The hull of the kernels may be broken by the animal via chewing, or may be mechanically broken by the kernel processor prior to being fed to the animal. Fracturing or cracking the hull improves the digestibility of the kernel, thereby allowing a greater amount of the nutrients within the kernel to be absorbed by the animal, and reducing waste of useful nutrients. The crop material is directed from the kernel processor into a discharge spout, which directs the crop material through an exit and into a storage container.

A maximum potential nutrient value of the crop material may be achieved when the crop material is processed to a desired degree or level of processing. Failure to process the crop material to these desired levels may result in the crop material failing to deliver its maximum potential nutrient value to an animal when consumed. The nutrients of the crop material that are not absorbed by the animal are wasted, thereby reducing the effectiveness and/or efficiency of the crop material as animal feed. Accordingly, it is desirable to process the crop material to the desired degree or level of processing to maximize nutrient absorption by the animal.

Different crop materials, or crop materials at different stages of maturation may require different amounts of processing and/or different settings for the crop processor to achieve the desired degree or level of processing to maximize nutrient absorption by the animal. For example, kernels of corn that are more mature tend to have a lower moisture content and have a drier, harder shell. Corn kernels having a lower moisture content, and thereby having a harder shell require a smaller roll gap between processing rolls of a kernel processor to achieve a desired level or kernel fracture or cracking, whereas corn kernels having a higher moisture content, and thereby having a softer shell may use a larger roll gap between the processing rolls to achieve the same level of kernel fracture or cracking.

Reference is made to the prior art described in DE 100 30 505 A1.

### SUMMARY

A harvester is provided. The harvester includes a head unit that is operable to gather crop material and direct the crop material along a flow path. A crop processor is positioned to receive the crop material from the head unit and partially defines the flow path of the crop material. The crop processor is operable to process the crop material to alter a characteristic of the crop material. A moisture sensor is operable to sense data related to a moisture content of the crop material as the crop material moves along the flow path. A computing device is in communication with the moisture sensor. The computing device includes a processor and a memory having a crop processing analysis algorithm stored thereon. The processor is operable to execute the crop processing analyses algorithm to receive the data related to the moisture content of the crop material from the moisture sensor. The computing device may then analyze the data related to the moisture content to determine an actual moisture content of the crop material. The computing device may then adjust the crop processor based on the actual moisture content of the crop material to achieve a desired level of alteration to the characteristic of the crop material.

In one aspect of the disclosure, the crop processor includes a kernel processor. The kernel processor includes a first processing roll and a second processing roll spaced from the first processing roll by a roll gap. The crop material passes through the roll gap between the first processing roll and the second processing roll for fracturing a kernel portion of the crop material. The processor is operable to execute the crop processing analyses algorithm to adjust the roll gap based on the actual moisture content of the crop material.

In one aspect of the disclosure, the processor is operable to execute the crop processing analyses algorithm to receive a plurality of user defined moisture ranges via a user input, such as but not limited to a touch screen display. Each of the user defined moisture ranges has a corresponding user defined processor setting. In one implementation, the user defined processor setting is a user defined roll gap for the kernel processor. The computing device may determine which one of the plurality of user defined moisture ranges the actual moisture content of the crop material is within, and adjust the crop processor to the corresponding user defined processor setting, e.g., the user defined roll gap setting.

In one aspect of the disclosure, the moisture sensor is positioned downstream of the crop processor along the flow path of the crop material. The harvester may include a discharge spout that is positioned to receive the crop material from the crop processor and partially define the flow path of the crop material. In one implementation, the moisture sensor is positioned in the discharge spout.

In one aspect of the disclosure, the processor is operable to execute the crop processing analyses algorithm to predict a moisture content immediately ahead of the head unit relative to a direction of travel of the head unit. The computing device may predict the moisture content based at least partially on the actual moisture content of the crop material previously processed. The computing device may then adjust the crop processor based on the predicted moisture content immediately ahead of the head unit to achieve the desired level of alteration to the characteristic of the crop material.

A method of controlling a harvester is also provided. The method includes determining an actual moisture content of crop material with a computing device. The computing device may then adjust a roll gap between a first processing roll and a second processing roll of a kernel processor based on the actual moisture content of the crop material.

In one aspect of the disclosure, a plurality of user defined moisture ranges are defined. Each of the plurality of user defined moisture ranges has a corresponding user defined roll gap setting that is also defined. The plurality of user defined moisture ranges and the corresponding user defined roll gap setting for each moisture range is saved in a memory of the computing device.

The computing device may determine which one of the plurality of user defined moisture ranges the actual moisture content of the crop material is within, and adjust the roll gap to the user defined roll gap setting corresponding with the user defined moisture range that the actual moisture range is within.

In one aspect of the disclosure, the computing device may predict a moisture content immediately ahead of the head unit relative to a direction of travel of the head unit based at least partially on the actual moisture content of the crop material. The computing device may then adjust the roll gap to the user defined roll gap setting corresponding with the user defined moisture range that the predicted moisture content immediately ahead of the head unit is within.

The harvesting implement and the method described herein enable the crop processor to be controlled based on the moisture content of the crop material. Because, in some situations, the amount of crop processing required to achieve a desired level of crop processing is strongly related to the moisture content of the crop material, controlling the crop processor based on the moisture content provides a simple and convenient process to automatically control the crop processor.

The above features and advantages and other features and advantages of the present teachings are readily apparent from the following detailed description of the best modes for carrying out the teachings when taken in connection with the accompanying drawings.

### BRIEF DESCRIPTION OF THE DRAWINGS

FIG. 1 is a schematic cut-away side view of a harvester.
FIG. 2 is a schematic plan view of the harvester.
FIG. 3 is a chart showing a user input including a plurality of moisture ranges and a respective processor setting for each moisture range.
FIG. 4 is a flowchart representing a method of controlling the harvester.

### DETAILED DESCRIPTION

Those having ordinary skill in the art will recognize that terms such as "above," "below," "upward," "downward," "top," "bottom," etc., are used descriptively for the figures, and do not represent limitations on the scope of the disclosure, as defined by the appended claims. Furthermore, the teachings may be described herein in terms of functional and/or logical block components and/or various processing steps. It should be realized that such block components may be comprised of any number of hardware, software, and/or firmware components configured to perform the specified functions.

Terms of degree, such as "generally", "substantially" or "approximately" are understood by those of ordinary skill to refer to reasonable ranges outside of a given value or orientation, for example, general tolerances or positional relationships associated with manufacturing, assembly, and use of the described embodiments.

Referring to the Figures, wherein like numerals indicate like parts throughout the several views, a harvester is generally shown at 20. The harvester 20 shown in the Figures and described herein is configured as a forage harvester. However, it should be appreciated that the harvester 20 may be configured differently than the example forage harvester shown in the Figures and described herein.

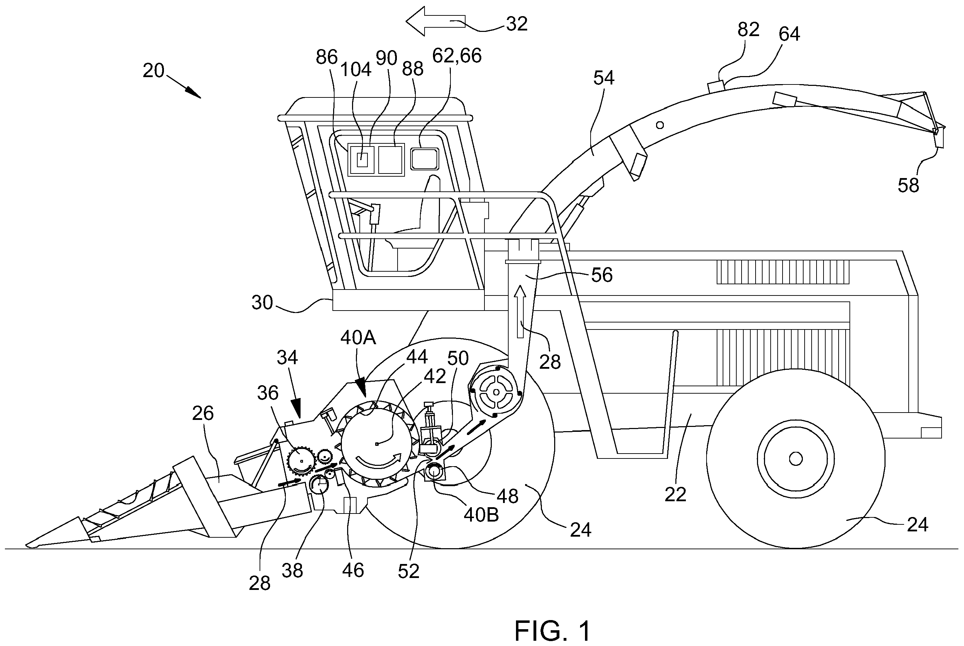

Referring to FIG. 1, the harvester 20 includes a frame 22, which supports the various components of the harvester 20. The frame 22 rotatably supports a plurality of ground engaging elements 24, such as but not limited to a pair of front wheels or tracks and a pair of rear wheels or tracks. In the example embodiment shown in FIG. 1 and described herein, the front wheels are drive wheels and the rear wheels are steerable wheels. However, it should be appreciated that the ground engaging elements 24 and the propulsion and steering thereof, may differ from the example embodiment shown in FIG. 1 and described herein.

Referring to FIG. 1, the harvester 20 includes a head unit 26. The head unit 26 is operable to gather crop material from a field and direct the crop material through the harvester 20 along a flow path 28. The head unit 26 is disposed at the forward end 30 of the harvester 20, relative to a direction of travel 32 of the harvester 20 when gathering the crop material. The head unit 26 is attached to and supported by the frame 22. The configuration and operation of the head unit 26 may vary depending upon the crop material being gathered. FIG. 1 shows an implementation of the head unit 26 operable for cutting and gathering standing corn. It should be appreciated that the head unit 26 may differ for other crop materials, such as alfalfa, grasses, sorghum, cereals, barley, fine grains, course grains, or other crop materials. The different configurations and operation of the different head units 26 are known to those skilled in the art, are not pertinent to the teachings of this disclosure, and are therefore not described in detail herein.

Referring to FIG. 1, the harvester 20 includes a feeder 34. The feeder 34 is positioned immediately rearward of the head unit 26 relative to the flow path 28 of the crop material. The feeder 34 is operable to move crop material gathered by the head unit 26 in a direction of crop processing and along the flow path 28. The direction of crop processing is generally directed rearward and possibly laterally relative to the direction of travel 32 of the harvester 20 when gathering crop material. In the example embodiment described herein, the feeder 34 may include a pair of opposing feed rollers, i.e., an upper feed roller 36 and a lower feed roller 38. The upper feed roller 36 and the lower feed roller 38 are spaced apart from each, with the gathered crop material fed between the upper feed roller 36 and the lower feed roller 38. The upper feed roller 36 and the lower feed roller 38 are counter-rotated relative to each other to move the crop material therebetween. The specific details and operation of the feeder 34 are known to those skilled in the art, are not pertinent to the teachings of this disclosure, and are therefore not described in greater detail herein. Furthermore, the configuration and operation of the feeder 34 may differ from the example embodiment shown in the Figures and described herein.

Referring to FIG. 1, the harvester 20 further includes at least one crop processor 40A, 40B. The crop processor 40A, 40B is disposed downstream of the feeder 34 relative to the direction of crop processing of the crop material. The crop processor 40A, 40B may include, but is not limited to, a cutter head 40A and/or a kernel processor 40B. The crop processor 40A, 40B is positioned to receive the crop material from the head unit 26 and partially define the flow path 28 of the crop material. In the example implementation described herein, the crop processor 40A, 40B receives the crop material from the head unit 26 via the feeder 34. The crop processor 40A, 40B is operable to process the crop material to alter a characteristic of the crop material. The characteristic of the crop material may include a physical, chemical, or nutritional property of one or more components of the crop material. For example, the characteristic of the crop material may include, but is not limited to, a length of stem portions of the crop material, a degree of fracture or cracking of the stem portions of the crop material, or a degree of fracture or cracking of kernel portions or a kernel wall of the crop material.

In the example implementation shown in the Figures, the cutter head 40A is positioned downstream of the feeder 34, relative to the flow path 28 of the crop material. The cutter head 40A is rotatably attached to the frame 22 and is rotatable about an axis of rotation. The axis of rotation of the cutter head 40A is generally perpendicular to the direction of travel 32 of the harvester 20 while gathering crop material, and generally perpendicular to the direction of crop processing. The example embodiment of the cutter head 40A shown in the Figures and described herein includes a cylindrical drum 42 having a plurality of knives 44 disposed circumferentially about the outer periphery of the drum 42.

Referring to FIG. 1, a shear bar 46 is located immediately downstream of the feeder 34 relative to the direction of crop processing of the crop material. The shear bar 46 is attached to and supported by the frame 22. The cutter head 40A cooperates with the shear bar 46 to cut the crop material into smaller pieces, with each of the smaller pieces having or defining a respective cut length. As such, the characteristic of the crop material may include the cut length of the stem portions of the crop material, with at least the feeder 34 and/or the cutter head 40A being operable to alter the cut length of the stem portions of the crop material. The drum 42 of the cutter head 40A rotates in a rotational direction about its axis of rotation, with the knives 44 oriented to cut the crop material when the drum 42 rotates. The shear bar 46 braces the crop material against the cutting action of the knives 44 to facilitate the cutting of the crop material. At least one of the shear bar 46 and the cylindrical drum 42 may move relative to the frame 22 such that the shear bar 46 and the cylindrical drum 42 may be moveable relative to each other to adjust the cut length of the crop material. The specific features and operation of the cutter head 40A and its relation to the shear bar 46 with regard to cutting the crop material to the cut length are known to those skilled in the art, are not pertinent to the teachings of this disclosure, and are therefore not described in greater detail herein.

Referring to FIG. 1, the kernel processor 40B is positioned downstream of the cutter head 40A relative to the flow path 28 of the crop material, and receives the crop material from the cutter head 40A. The kernel processor 40B includes a pair of opposing processing rolls, i.e., a first processing roll 48 and a second processing roll 50. The first processing roll 48 and the second processing roll 50 are rotated at different speeds to further process portions of the crop material, e.g., kernels of the crop material, by fracturing or cracking one or more walls of the kernels. As such, the characteristic of the crop material may include the wall of the kernels, i.e., the kernel wall, with the kernel processor 40B being operable to crack or fracture the kernel wall of the kernels of the crop material. As used herein, the term "kernel wall" includes the bran layer of a grain. As understood by those skilled in the art, the bran layer is the hard outer layer of a grain that protects the seed. The first processing roll 48 and the second processing roll 50 are separated by a roll gap 52 and are biased together. The roll gap 52 may be between, approximately, 0.75 mm and 3.0 mm. At least one of the first processing roll 48 and the second processing roll 50 is moveable relative to the frame 22, such that the first processing roll 48 and the second processing roll 50 are moveable relative to each other to adjust the distance of the roll gap 52 for different crop materials. Each of the first processing roll 48 and the second processing roll 50 may include teeth, ridges, valleys, etc., that help fracture and/or crack the kernel walls of the kernel portions of the crop material to improve digestibility. The specific features and operation of the kernel processor 40B are known to those skilled in the art, are not pertinent to the teachings of this disclosure, and are therefore not described in greater detail herein.

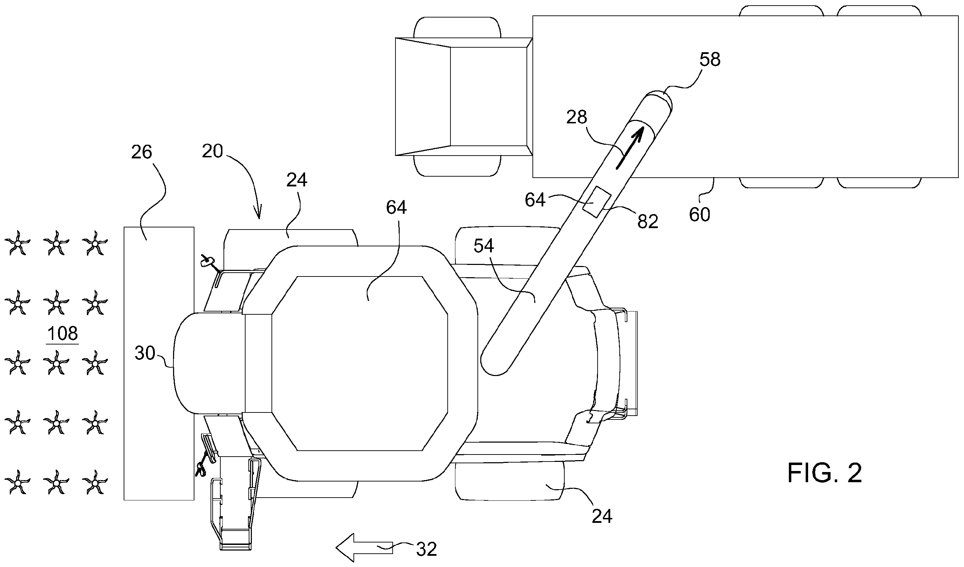

The harvester 20 further includes a discharge spout 54. The discharge spout 54 is positioned downstream of the kernel processor 40B relative to the flow path 28 of the crop material. The discharge spout 54 includes an inlet 56 positioned to receive the crop material from the crop processor 40A, 40B, e.g., the cutter head 40A and/or the kernel processor 40B, and partially defines the flow path 28 of the crop material. The discharge spout 54 may include an exit 58 that is positioned to expel the crop material into a storage container 60. The discharge spout 54 may include, but is not limited to, an elongated tubular structure that is shaped to guide and direct the crop material into the storage container 60. In one implementation, the storage container 60 may include a bin supported by the frame 22 and integral with the harvester 20. In another implementation, such as shown in FIG. 2, the storage container 60 may include a truck, trailer, dump truck, semi-truck and trailer, or other similar vehicle and/or vehicle trailer combination that is positioned adjacent to the harvest implement and positioned to receive the crop material from the discharge spout 54.

The harvester includes a moisture sensor 64 that is operable to sense data related to a moisture content of the crop material as the crop material moves along the flow path 28. In the example implementation shown in the Figures and described herein, the moisture sensor 64 is positioned downstream of the crop processor 40A, 40B along the flow path 28 of the crop material. For example, the moisture sensor 64 may be positioned in the discharge spout 54. However, in other implementations, the moisture sensor 64 may be positioned upstream of the crop processor 40A, 40B along the flow path 28 of the crop material.

The moisture sensor 64 may include any device that is capable of sensing data related to the moisture content of the crop material. For example, the harvester 20 may include a Near InfraRed (NIR) sensor 82 that is positioned to capture a NIR image of the crop material in a NIR light spectrum. The NIR sensor 82 may be positioned at any point along the flow path 28 of the crop material. In the example implementation shown in the Figures and described herein, the NIR sensor 82 is positioned within a wall 84 of the discharge spout 54 to capture the NIR image of the crop material within the discharge spout 54. The NIR sensor 82 captures the NIR image in the NIR light spectrum. The NIR light spectrum includes light having a wavelength between the range of approximately 700 nanometers and 2,500 nanometers. The NIR image may be analyzed by the NIR sensor 82 or by a computing device 86 (described below) to determine the moisture content of the crop material. It should be appreciated that the moisture sensor 64 may be implemented in other ways using other systems and/or sensing devices that sense other types of data related to the moisture content of the crop material.

Referring to FIG. 1, the harvester 20 may further include the computing device 86. The computing device 86 is disposed in communication with the moisture sensor 64 and the crop processor 40A, 40B. The computing device 86 may alternatively be referred to as a computer, a controller, a control unit, a control module, etc. The computing device 86 may be located on the harvester 20, or remote from the harvester 20. The computing device 86 is operable to monitor the operation of the crop processor 40A, 40B, and may additionally be operable to control the operation of the harvester 20. The computing device 86 includes a processor 88, a memory 90, and all software, hardware, algorithms, connections, sensors, etc., necessary to monitor and/or control the operation of the one or more components of the harvester 20, such as but not limited to, the crop processor 40A, 40B, etc. As such, a method may be embodied as a program or algorithm operable on the computing device 86. It should be appreciated that the computing device 86 may include any device capable of analyzing data from various sensors, comparing data, making the necessary decisions required to monitor and/or control the operation of the crop processor 40A, 40B, or some other component of the harvester 20.

As used herein, "computing device" or "controller" are intended to be used consistent with how the term is used by a person of skill in the art, and refers to a computing component with processing, memory, and communication capabilities, which is utilized to execute instructions (i.e., stored on the memory 90 or received via the communication capabilities) to control or communicate with one or more other components. In certain embodiments, a controller may also be referred to as a control unit, vehicle control unit (VCU), engine control unit (ECU), transmission control unit (TCU), or electrical controller. In certain embodiments, a controller may be configured to receive input signals in various formats (e.g., hydraulic signals, voltage signals, current signals, CAN messages, optical signals, radio signals), and to output command or communication signals in various formats (e.g., hydraulic signals, voltage signals, current signals, CAN messages, optical signals, radio signals).

The computing device 86 may be in communication with other components on the harvester 20, such as hydraulic components (e.g., valve block), electrical components (e.g., solenoid, accumulator sensor), actuators, sensors, and operator inputs within an operator station of the work vehicle. The computing device 86 may be electrically connected to these other components by a wiring harness such that messages, commands, and electrical power may be transmitted between the computing device 86 and the other components. Although the computing device 86 is referenced in the singular, in alternative implementations the configuration and functionality described herein can be split across multiple computing devices using techniques known to a person of ordinary skill in the art.

The computing device 86 may be embodied as one or multiple digital computers or host machines each having one or more processors, read only memory (ROM), random access memory (RAM), electrically-programmable read only memory (EPROM), optical drives, magnetic drives, etc., a high-speed clock, analog-to-digital (A/D) circuitry, digital-to-analog (D/A) circuitry, and any required input/output (I/O) circuitry, I/O devices, and communication interfaces, as well as signal conditioning and buffer electronics.

The computer-readable memory 90 may include any non-transitory/tangible medium which participates in providing data or computer-readable instructions. The memory 90 may be non-volatile or volatile. Non-volatile media may include, for example, optical or magnetic disks and other persistent memory. Example volatile media may include dynamic random access memory (DRAM), which may constitute a main memory. Other examples of embodiments for memory include a floppy, flexible disk, or hard disk, magnetic tape or other magnetic medium, a CD-ROM, DVD, and/or any other optical medium, as well as other possible memory devices such as flash memory.

As described above, the computing device 86 includes the processor 88 and the memory 90. The memory 90 includes a crop processing analysis algorithm 104 stored thereon. The processor 88 is operable to execute the crop processing analysis algorithm 104 to implement a method of monitoring the operation of the crop processor 40A, 40B, and/or controlling the harvester 20.

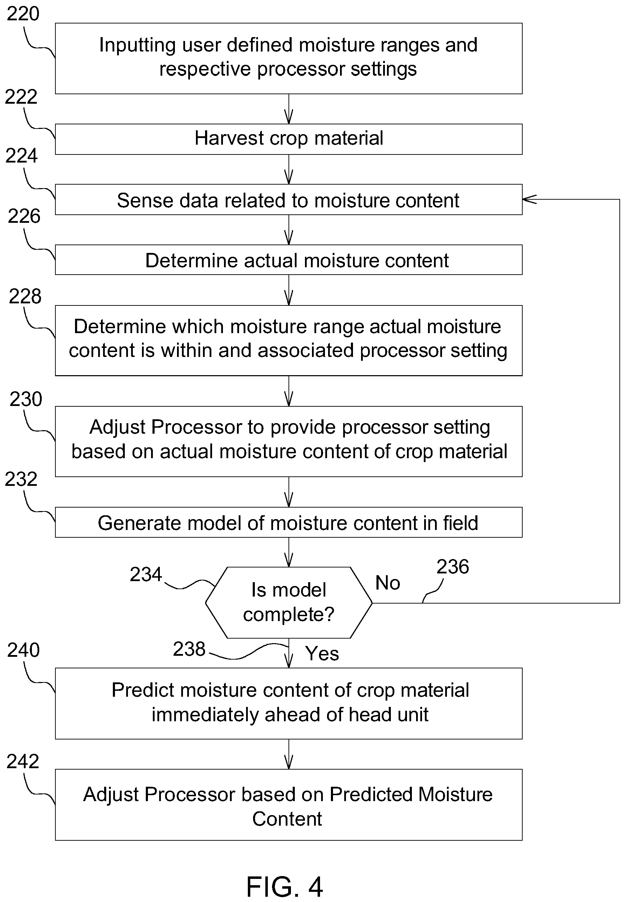

Referring to FIG. 4, the processor 88 is operable to execute the crop processing analysis algorithm 104 to receive a user input providing a plurality of user defined moisture ranges 68, 70, 72, 74, 76. The step of inputting the user defined moisture ranges 68, 70, 72, 74, 76 and their respective processor settings is generally indicated by box 220 shown in FIG. 4. Each of the user defined moisture ranges 68, 70, 72, 74, 76 has a corresponding user defined processor setting. Referring to FIG. 1, the user defined moisture ranges 68, 70, 72, 74, 76 and their respective user defined processor settings may be input by an operator into the computing device 86 using a suitable input device 66, such as but not limited to, a keyboard, a touch screen display, audio receiver, joystick, etc. The input device 66 may be separate from, or integral with an output 62. For example, the input device 66 and the output 62 may be combined as a touch screen display or other similar device located in a cab of the harvester 20.

The actual moisture content of the crop material may be related to and/or correspond with a setting for the crop processor 40A, 40B that is necessary to achieve a desired level of crop processing. In other words, the settings for the crop processor 40A, 40B in order to achieve the desired level of alteration to the crop material or a desired level or crop processing may change dependent upon the actual moisture content of the crop material. For example, kernel portions of crop material having a higher actual moisture content are relatively easier to crack or fracture when compared to kernel portions of crop material having a lower actual moisture content. As such, in order to achieve a desired level of crop processing, e.g., a specific level of kernel wall fracture or cracking, the roll gap 52 of the kernel processor 40B may be set to a smaller distance when processing kernels having a higher moisture content, and may need to be set to a larger distance when processing kernels having a lower moisture content. Accordingly, the roll gap 52 for the kernel processor 40B required to achieve a certain level of kernel fracture varies or changes in relation to the moisture content of the crop material. The process described herein adjusts the crop processor 40A, 40B based on the actual moisture content of the crop material to achieve a desired level of alteration to the characteristic of the crop material.

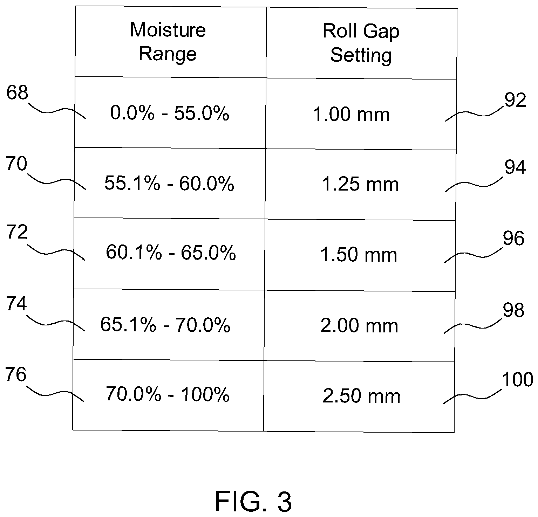

The number or moisture ranges 68, 70, 72, 74, 76 may include two ranges, three ranges, four ranges, etc. Each of the moisture ranges 68, 70, 72, 74, 76 may be defined to include a lower limit and an upper limit. For example, referring to FIG. 3, a total of five example moisture ranges 68, 70, 72, 74, 76 are defined, with each of the respective moisture ranges 68, 70, 72, 74, 76 having a defined processor setting. It should be appreciated that the moisture ranges 68, 70, 72, 74, 76 may differ from the example number and value of the ranges shown in FIG. 3.

As shown in FIG. 3, a first range 68 is defined to include a lower moisture limit of 0.0%, and an upper moisture limit of 55.0%. A second range 70 is defined to include a lower moisture limit of 55.1% and an upper moisture limit of 60.0%. A third range 72 is defined to include a lower moisture limit of 60.1% and an upper moisture limit of 65.0%. A fourth range 74 is defined to include a lower moisture limit of 65.1% and an upper moisture limit of 70.0%. A fifth range 76 is defined to include a lower moisture limit of 70.1% and an upper moisture limit of 100%.

In the example implementation shown in FIG. 3, the defined processor setting for each respective one of the plurality of user defined moisture ranges 68, 70, 72, 74, 76 is a user defined roll gap setting for the kernel processor. However, in other implementations, the defined processor setting may include a different processor setting, such as but not limited to a cut length setting for the cutter head. In the example implementation shown in FIG. 3, the first range 68 has a respective first roll gap setting 92 that is equal to 1.00 mm. The second range 70 has a respective second roll gap setting 94 that is equal to 1.25 mm. The third range 72 has a respective third roll gap setting 96 that is equal to 1.50 mm. The fourth range 74 has a respective fourth roll gap setting 98 that is equal to 2.00 mm. The fifth range 76 has a respective fifth roll gap setting 100 that is equal to 2.50 mm.

Once the user defined moisture ranges 68, 70, 72, 74, 76 and their respective user defined processor settings have been input into the computing device 86, operation of the harvester 20 may begin. The step of harvesting the crop material is generally indicated by box 222 shown in FIG. 4. It should be appreciated that operation of the harvester 20 includes maneuvering the harvester 20 through a field, whereby the head unit 26 gathers the crop material from the field. Once the crop material is gathered, the feeder 34 moves the crop material in the direction of crop processing along the flow path 28 of the crop material. In the example implementation of the harvester 20 shown in the figures and described herein, the crop material moves through the cutter head 40A, whereby the stem portions of the crop material are cut to define an actual cut length of the stem portions. Following the cutter head 40A, the crop material moves through the kernel processor 40B, whereby the walls of the kernel portions of the crop material are fractured or cracked. As noted above, processing the crop material, i.e., cutting the stems and/or fracturing or cracking the kernels, improves the digestibility of the crop material, thereby allowing a greater amount of the nutrients within the crop material to be absorbed by the animal, and reducing waste of useful nutrients. Upon exiting the kernel processor 40B, the crop material moves through the entrance of the discharge spout 54. The discharge spout 54 directs the crop material therethrough and dispenses the crop material through the exit 58 of the discharge spout 54, into the storage container 60.

In the example implementation of the harvester 20 described herein, as the crop material moves through the discharge spout 54, the processor 88 is operable to execute the crop processing analysis algorithm 104 to sense data related to the moisture content of the crop material with the moisture sensor 64. The step of sensing the data related to the actual moisture content of the crop material is generally indicated by box 224 shown in FIG. 4. The moisture sensor 64 may then communicate the data related to the moisture content of the crop material to the computing device 86. As described above, the moisture sensor 64 may be implemented with the NIR sensor 82 described herein. The NIR sensor 82 may continuously capture NIR images, and communicate the NIR images to the computing device 86 for analysis.

The computing device 86 is operable to receive the data related to the moisture content of the crop material from the moisture sensor 64. For example, in the implementation described herein, the computing device 86 may receive the NIR images from the NIR sensor 82. However, in other implementations, the data related to the moisture content of the crop material received from the moisture sensor 64 may differ from the example NIR images described herein.

Once the computing device 86 has received the data related to the moisture content of the crop material from the moisture sensor 64, the computing device 86 may analyze the data related to the moisture content to determine an actual moisture content of the crop material. The step of determining the actual moisture content is generally indicated by box 226 shown in FIG. 4. The way the computing device 86 may analyze the data related to the moisture content may differ or vary based on the type and/or form of the data. In the example implementation described herein, the processor 88 may be operable to execute the crop processing analysis algorithm 104 to analyze the NIR image to determine the moisture content and/or a starch content of the crop material. For example, the computing device may include image and/or color recognition and analysis software that identifies the objects and their respective color in the near infrared color spectrum, and associates those colors with a respective moisture content. It should be appreciated that the computing device 86 may analyze the NIR image to determine the moisture content of the crop material in some other manner not described herein. Furthermore, it should be appreciated that the analysis of the NIR image may occur within the NIR sensor 82 itself, and that the data related to the moisture content of the crop material communicated to the computing device 86 from the NIR sensor 82 may include the actual moisture content percentage of the crop material.

Once the moisture content of the crop material has been determined, the processor 88 is operable to execute the crop processing analyses algorithm 104 to determine which one of the plurality of user defined moisture ranges 68, 70, 72, 74, 76 the actual moisture content of the crop material is within. The step of determining which of the plurality of moisture ranges 68, 70, 72, 74, 76 and the associated processor setting for the actual moisture content is generally indicated by box 228 shown in FIG. 4. For example, referring to FIG. 3, in the example implementation described herein, if the actual moisture content of the crop material is determined to be 63.0%, then the computing device 86 may determine that the actual moisture content of the crop material is within the third range 72.

As noted above, each of the different user defined moisture ranges 68, 70, 72, 74, 76 has a respective user defined processor setting. Once the computing device has determined which one of the plurality of user defined moisture ranges 68, 70, 72, 74, 76 the actual moisture content of the crop material is within, then the computing device may adjust the crop processor 40A, 40B to the corresponding user defined processor setting. The step of adjusting the crop processor 40A, 40B to provide the user defined processor setting is generally indicated by box 230 shown in FIG. 4. In the example implementation described herein, the user defined processor setting is a user defined roll gap setting 92, 94, 96, 98, 100. Accordingly, in the example implementation described herein, the processor 88 is operable to execute the crop processing analyses algorithm 104 to adjust the roll gap 52 to achieve the roll gap setting 92, 94, 96, 98, 100 based on the actual moisture content of the crop material.

Because the roll gap setting 92, 94, 96, 98, 100 is related to the desired level of alteration to the characteristic of the crop material, e.g., the percentage or actual degree of kernel wall fracture or cracking, adjusting the roll gap 52 based on the actual moisture content of the crop material enables the harvester 20 to achieve the desired level of alteration of the characteristic of the crop material, e.g., the percentage or actual degree of kernel wall fracture or cracking of the crop material.

As described above, the process of controlling the harvester 20 uses the actual moisture content of harvested crop material to retroactively control the processor setting, e.g., the roll gap 52 of the kernel processor 40B. However, it is contemplated that the actual moisture content of the harvested crop material may be tracked throughout the field, thereby providing sufficient data for the computing device 86 to develop a model predicting the moisture content throughout the remainder of the field. The step of developing the model of the moisture content in the field is generally indicated by box 232. As such, the processor 88 may be operable to execute the crop processing analyses algorithm to predict a moisture content immediately ahead of the head unit 26 relative to a direction of travel 32 of the head unit 26 based at least partially on the actual moisture content of the crop material previously processed. The computing device 86 may use the actual moisture content from crop material, in combination with other data, such as but not limited to previously harvested crop data, weather data, etc., and generate the map or model of predicted moisture content throughout the field.

The computing device 86 may determine if the model is complete, or is not complete. the step of determining if the model is complete is generally indicated by box 234 shown in FIG. 4. If the computing device 86 determines that the model is not complete, generally indicated at 236 in FIG. 4, then the process continues to control the crop processor 40A, 40B, retroactively based on the actual moisture content of the crop material previously harvested.

If the computing device 86 determines that the model is complete, generally indicated at 238 in FIG. 4, then the computing device may use the map of the predicted moisture content to determine a predicted moisture content of the crop material 108 immediately ahead of the head unit 26. The step of predicting the moisture content of the crop material from the model is generally indicated by box 240 shown in FIG. 4. The process, techniques, and software for generating the model of predicted moisture content of the crop material are known to those skilled in the art, and are therefore not described in detail herein.

Once the computing device 86 has generated the model of the predicted moisture content of the crop material, based at least in part on the actual moisture content of the crop material previously harvested that day, the computing device 86 may then adjust the crop processor 40A, 40B based on the predicted moisture content immediately ahead of the head unit 26 to achieve the desired level of alteration to the characteristic of the crop material. The step of adjusting the processor setting based on the predicted moisture content of the crop material from the model derived from the actual moisture content of the crop material is generally indicated by box 242 shown in FIG. 4. For example, and as described above, the computing device 86 may adjust the roll gap 52 to provide the defined roll gap setting 92, 94, 96, 98, 100 for the predicted moisture content of the crop material. In doing so, the computing device 86 may determine which moisture range the predicted moisture content of the crop material 108 immediately ahead of the heat unit 26 is within, and then control the control the crop processor 40A, 40B to provide the processor setting, e.g., roll gap setting 92, 94, 96, 98, 100 associated with that respective moisture range. In so doing, the computing device 86 may proactively control the crop processor 40A, 40B, e.g., the roll gap 52 of the kernel processor 40B, based on the actual moisture content of the crop material, because the model used to generate the predicted moisture content of the crop material is based on the actual moisture content of the crop material measured at that time.

As used herein, "e.g." is utilized to non-exhaustively list examples, and carries the same meaning as alternative illustrative phrases such as "including," "including, but not limited to," and "including without limitation." As used herein, unless otherwise limited or modified, lists with elements that are separated by conjunctive terms (e.g., "and") and that are also preceded by the phrase "one or more of," "at least one of," "at least," or a like phrase, indicate configurations or arrangements that potentially include individual elements of the list, or any combination thereof. For example, "at least one of A, B, and C" and "one or more of A, B, and C" each indicate the possibility of only A, only B, only C, or any combination of two or more of A, B, and C (A and B; A and C; B and C; or A, B, and C). As used herein, the singular forms "a", "an" and "the" are intended to include the plural forms as well, unless the context clearly indicates otherwise. Further, "comprises," "includes," and like phrases are intended to specify the presence of stated features, steps, operations, elements, and/or components, but do not preclude the presence or addition of one or more other features, steps, operations, elements, components, and/or groups thereof.

## Claims

1. A harvester comprising:
a head unit (26) operable to gather crop material and direct the crop material along a flow path (28);
a crop processor (40A, 40B) positioned to receive the crop material from the head unit (26) and partially define the flow path (28) of the crop material, wherein the crop processor (40A, 40B) is operable to process the crop material to alter a characteristic of the crop material;
a moisture sensor (64) operable to sense data related to a moisture content of the crop material as the crop material moves along the flow path (28);
a computing device in communication with the moisture sensor (64) and including a processor (88) and a memory (90) having a crop processing analysis algorithm (104) stored thereon, wherein the processor (88) is operable to execute the crop processing analysis algorithm (104) to:
receive the data related to the moisture content of the crop material from the moisture sensor (64);
analyze the data related to the moisture content to determine an actual moisture content of the crop material; and
adjust the crop processor (40A, 40B) based on the actual moisture content of the crop material to achieve a desired level of alteration to the characteristic of the crop material.

2. The harvester set forth in claim 1, wherein the crop processor (40A, 40B) includes a kernel processor (40B) having a first processing roll (48) and a second processing roll (50) spaced from the first processing roll (48) by a roll gap (52), and wherein the crop material passes through the roll gap (52) between the first processing roll (48) and the second processing roll (50) for fracturing a kernel portion of the crop material.

3. The harvester set forth in claim 1 or 2, wherein the processor (88) is operable to execute the crop processing analysis algorithm (104) to adjust the roll gap (52) based on the actual moisture content of the crop material.

4. The harvester according to at least one of the claims 1 to 3, wherein the processor (88) is operable to execute the crop processing analysis algorithm (104) to receive a plurality of user defined moisture ranges, with each of the plurality of user defined moisture ranges having a corresponding user defined processor setting.

5. The harvester according to at least one of the claims 1 to 4, wherein the processor (88) is operable to execute the crop processing analysis algorithm (104) to determine which one of the plurality of user defined moisture ranges the actual moisture content of the crop material is within, and adjust the crop processor (40A, 40B) to the corresponding user defined processor setting.

6. The harvester according to at least one of the claims 1 to 5, wherein the crop processor (40A, 40B) includes a kernel processor (40B) having a first processing roll (48) and a second processing roll (50) spaced from the first processing roll (48) by a roll gap (52), and wherein the crop material passes through the roll gap (52) between the first processing roll (48) and the second processing roll (50) for fracturing a kernel portion of the crop material.

7. The harvester according to at least one of the claims 1 to 6, wherein the user defined processor setting for each of the plurality of moisture ranges is a user defined roll gap (52) for the kernel processor (40B).

8. The harvester according to at least one of the claims 1 to 7, wherein the moisture sensor (64) is positioned downstream of the crop processor (40A, 40B) along the flow path (28) of the crop material.

9. The harvester according to at least one of the claims 1 to 8, further comprising a discharge spout (54) positioned to receive the crop material from the crop processor (40A, 40B) and partially define the flow path (28) of the crop material.

10. The harvester according to at least one of the claims 1 to 9, wherein the moisture sensor (64) is positioned in the discharge spout (54).

11. The harvester according to at least one of the claims 1 to 10, wherein the processor (88) is operable to execute the crop processing analysis algorithm (104) to predict a moisture content immediately ahead of the head unit (26) relative to a direction of travel (32) of the head unit (26) based at least partially on the actual moisture content of the crop material previously processed, and adjust the crop processor (40A, 40B) based on the predicted moisture content immediately ahead of the head unit (26) to achieve the desired level of alteration to the characteristic of the crop material.

## Amended claims

### Amended claims in accordance with Rule 137(2) EPC.

1. A harvester comprising:
a head unit (26) operable to gather crop material (108) and direct the crop material (108) along a flow path (28);
a crop processor (40A, 40B) positioned to receive the crop material (108) from the head unit (26) and partially define the flow path (28) of the crop material (108), wherein the crop processor (40A, 40B) is operable to process the crop material (108) to alter a characteristic of the crop material (108);
a moisture sensor (64) operable to sense data related to a moisture content of the crop material (108) as the crop material (108) moves along the flow path (28);
a computing device (68) in communication with the moisture sensor (64) and including a processor (88) and a memory (90) having a crop processing analysis algorithm (104) stored thereon, wherein the processor (88) is operable to execute the crop processing analysis algorithm (104) to receive the data related to the moisture content of the crop material (108) from the moisture sensor (64); analyze the data related to the moisture content to determine an actual moisture content of the crop material (108); and adjust the crop processor (40A, 40B) based on the actual moisture content of the crop material (108) to achieve a desired level of alteration to the characteristic of the crop material (108);
**characterized in that** the processor (88) is configured to execute the crop processing analysis algorithm (104) to predict a moisture content immediately ahead of the head unit (26) relative to a direction of travel (32) of the head unit (26) based on a map of predicted moisture content throughout the field, the map generated at least partially on the actual moisture content of the crop material (108) previously processed throughout the field, and adjust the crop processor (40A, 40B) based on the predicted moisture content immediately ahead of the head unit (26) to achieve the desired level of alteration to the characteristic of the crop material (108).

2. The harvester set forth in claim 1, wherein the crop processor (40A, 40B) includes a kernel processor (40B) having a first processing roll (48) and a second processing roll (50) spaced from the first processing roll (48) by a roll gap (52), and wherein the crop material (108) passes through the roll gap (52) between the first processing roll (48) and the second processing roll (50) for fracturing a kernel portion of the crop material (108).

3. The harvester set forth in claim 2, wherein the processor (88) is operable to execute the crop processing analysis algorithm (104) to adjust the roll gap (52) based on the actual moisture content of the crop material (108).

4. The harvester according to at least one of the claims 1 to 3, wherein the processor (88) is operable to execute the crop processing analysis algorithm (104) to receive a plurality of user defined moisture ranges, with each of the plurality of user defined moisture ranges having a corresponding user defined crop processor setting.

5. The harvester according to claim 4, wherein the processor (88) is operable to execute the crop processing analysis algorithm (104) to determine which one of the plurality of user defined moisture ranges the actual moisture content of the crop material (108) is within, and adjust the crop processor (40A, 40B) to the corresponding user defined crop processor setting.

6. The harvester according to claim 4 or 5, when appended to claim 3, wherein the user defined crop processor setting for each of the plurality of moisture ranges is a user defined roll gap (52) for the kernel processor (40B).

7. The harvester according to at least one of the claims 1 to 6, wherein the moisture sensor (64) is positioned downstream of the crop processor (40A, 40B) along the flow path (28) of the crop material (108).

8. The harvester according to at least one of the claims 1 to 7, further comprising a discharge spout (54) positioned to receive the crop material (108) from the crop processor (40A, 40B) and partially define the flow path (28) of the crop material (108).

9. The harvester according to claim 8, wherein the moisture sensor (64) is positioned in the discharge spout (54).
